# EUROPEAN PATENT SPECIFICATION

(11) **EP 2 594 217 B1**
(45) Date of publication and mention of the grant of the patent: **12.04.2017**
(21) Application number: 11806160.5
(22) Date of filing: 06.07.2011
(51) Int. Cl.: A61B 90/00, A61B 17/04, A61B 17/82, A61B 17/88

(54) **SHEARS WITH TENSIONER AND DYNAMOMETER DEVICE**
SCHERE MIT EINEM SPANNER UND EINER DYNAMOMETERVORRICHTUNG
CISAILLE AVEC DISPOSITIF TENDEUR ET DYNAMOMÈTRE

(30) Priority: 12.07.2010 BR PI1002494
(43) Date of publication of application: 22.05.2013
(73) Proprietor: De Oliveira, Joao Bosco, 05657-230 Sao Paulo (BR)
(72) Inventor: De Oliveira, Joao Bosco, 05657-230 Sao Paulo (BR)
(74) Representative: Fernández-Pacheco, Aurelio Fernández
(86) International application number: PCT/BR2011/000210
(87) International publication number: WO 2012/006699

(56) References cited:
- EP-A1- 0 597 258
- EP-A1- 1 634 540
- EP-A1- 1 731 109
- WO-A1-2005/094708
- US-A- 5 312 410
- US-A1- 2004 199 169
- US-A1- 2005 240 198

## Description

### APPLICATION FIELD

The present patent application described and claimed herein is directed to a new solution in ancillary tool for the application of a metal clamping element designed to fasten two or more portions of parts, objects, products, being specially applied for promoting the attachment of split bone structures every time an invasive surgical intervention takes place in elective medical procedures or also when some bone structures present spontaneous or accidental factures, thus assuring an adequate mandatory recovery of the affected area, as well as for the fixation of plates or connecting rods that may assist in the stabilization of such fractures.

### DISCLOSURE OF THE INVENTION

After having defined manufacturing and selling of products for surgical purposes, specially for products aimed at invasive surgeries, the inventor has identified the need to provide a surgical team with a device that may assure a quick way to handle and fix metal clamps, specially metal clamps applied to assist in the consolidation of the junction of at least two edges of a bone structure where the invasive surgical intervention has been carried out, an example of which includes mandatory ruptures in the breastbone when a cardiovascular surgery takes place. In addition, this requirement is extended to a condition where the surgical team comes across with a spontaneous or traumatic bone fracture.

The surgical procedure point of view: when invasive surgical procedures are carried out, especially those that last long, those skilled in the medical area specialized in surgical procedures state that the stabilization of the involved parts in a fracture is imperative so that an adequate consolidation and reduction of the infection rate is attained, since this is one of the factors responsible for such occurrence.

The post-surgical procedure point of view: Again based on the expertise of those skilled in surgical procedures, the inventor has identified the new need to promote more improvement in the post-surgery general state of patients who had been submitted to surgical intervention. Although there are some improvement aspects to be considered in the monitoring and recovery of patients recently submitted to surgery, mainly cardiac patients, the inventor has established, as the main object of the present invention, the optimization of such recovery, the paradigms of which are:
1. The technical aspect: there is a mandatory need to attain higher quality in the application of clamps designed to consolidate the junction and scarring of two portion separated from bone structures when the surgical procedure is completed.
   Also, in this context, the surgeon and his/her assistant should carry out the procedure of applying said clamp, or set of clamps (in the event the breastbone is closed) with the utmost safety, that is, anticipating and preventing occasional accidents such as striking of the clamp application tool when the clamp itself is cut and affixed.
2. The patient health aspect: based on scrupulous in site studies on the evolution of the bone scarring when monitoring a patient in the hospital, it was noticed that the degree of suffering of a number of such patients is quite evident, with severe pain in the area of the breast where the mandatory incision of the "breastbone" has taken place.

In the study that required the conception of the present invention, the high degree of suffering the patient is subjected to after being submitted to a surgery is relevant, in other words it always exceeds tolerance peaks when the patient involuntarily coughs, a reaction that is quite frequent in cases of patients submitted to cardiac surgery.

### BACKGROUND OF THE INTENTION

In order to corroborate the context provided in the introduction, a brief explanation of techniques, devices and instruments usually used in the invasive surgical medicine area shall be provided, so that one skilled in the art specialized in the present subject matter can recognize the limiting aspects thereof by using the technical inceptions of the inventor, in order to provide the advantages attained with the shears with tensioner and dynamometer used in the application of surgical steel clamps.

The invasive surgical technique: assuming the cardiac surgery as an example it can be realized that it causes a painful traumatism recovery according to the patient point of view due to the fact that it is a invasive technique, thus requiring the need to carry out the incision of the bone known as "breastbone".

First of all, the invasive surgery requires the use of special instruments, and said instruments should be suitable for cutting and separating tissues (scalpels, scissors, forceps, splitters, clamps and saws) for carrying out the invasive intervention.

On its turn, for the reverse procedure, that is, the procedure that is used to repair structures that had undergone some trauma during the surgical intervention, the medical team make use of elements such as needles, needle holders, suture threads and/or ribbons, clamps, among others, which can or cannot be reabsorbed by the human organism.

The operational ergonomics: in surgical procedures, a medical team mandatorily works under borderline conditions when the time is constantly controlled and is thus a relevant parameter, since such conditions have caused the development of a specific unit to be applied in the surgical medicine field, specifically focused on the procedure for correcting bone fractures caused by any medical act (sternotomy), or accidentally or spontaneously acquired, and finally the procedure of rearranging the structures affected by the same should understood.

Additionally, it is pertinent to point out that in the area of cardiac surgery a unique operational ergonomics condition is mandatory when the bone structure known is closed, for example, the "breastbone", since the intervention herein is characterized by being quite aggressive, and ergonomics should be understood as the easy way in which the medical team carries out the procedure of affixing the "breastbone" into the spaces between the ribs, and such fixation is usually carried out by using steel threads and special clamps, mainly made of highly resistant histocompatible metal alloys.

### Instruments for applying clamps:

1. Conventional pliers: in order to apply said clamps, the medical team makes use of a number of instruments, predominantly by using cutting pliers, and it may be better defined as a king of pincers or tongs suitable to hold, grasp or cut certain objects, whose constructive concept brings about two components known as levers (made of iron or steel) that are mounted crossed over each other and rotate around an axle in the region they are connected together, the ends of which may be smooth or serrated, flat, curved, cylindrical or in the form of a bridge, amongst other forms.
   The critical analysis of these classic pliers when used for surgical procedures presents several limitations, mainly a limited ergonomics of use, since the grasping function is not dynamic, that is, since the clamp is fixed on the junction region of the bone structure, it is evident that the required tensioning in the junction region is not attained, thus generating gaps whose negative effect are potentiated when the patient is recovering from a surgery, mainly when this imposes breast distention movements when some "cough" occurs, which as a matter of fact is quite common in post-surgical procedures of this nature.
   According to the above, those skilled in the area of surgical medicine recognize unanimously that the use of conventional pliers is impracticable and unsuitable in view of the complexity and smoothness that are characteristic of an invasive procedure, since it compromises the consolidation of an invasive surgery.
   Also, it is evident that the lack of precision in the application of clamps, with the occurrence of excess gaps, derives from the fact that conventional pliers do not provide the medical team with the required degree of precision and agility in the application of the clamp set.
   Lastly, said pliers also present a prohibitive limitation as to its functionality as a cutting metal clamp, since it requires that the doctor or his/her assistant make a mandatory movement and effort deprived of ergonomics to maintain a tension close to the one desired at the end of the fixation of the clamp, thus adding handling difficulties and consequently incrementing the time for cutting the excess of the handle of said clamp.
2. The multi-function surgical pliers: recently, the state of the art has been improved by a new tool to be called multi-function surgical pliers designed for affixing metal clamps the purpose of which is to solve the restrictive aspects of the procedure of consolidating the invasive surgical procedure referred to in the previous sub-item, so that applying, tensioning, cutting and finishing functions for fixing the clamp are added to the same product by said tool.

The constructive concept of the multi-function pliers is comprised of the components:
- a first lever, provided at its upper end with a head designed to accommodate the excess segment of the metal clamp;
- a second lever provided with a housing for the spindle associated with the traction pin;
- a traction pin the function of which is to fix the end of the excess segment of the metal clamp;
- a spindle, to which the traction pin that assures the linear displacement of the traction pin is fixed, and is mounted inside the housing provided for in the second lever;
- a handle that provides rotation around its central axis and assures the displacement movement of the spindle and traction pin assembly; and

Also, the functional concept provided for in the multi-function pliers may comprise a four-step process, viz:
- first step: positioning of the metal clamp with its excess end adjusted close to the head specially designed for this product in such a way that said end fits into the traction pin after said adjustment;
- second step: tensioning of the clamp, wherein as this traction pin moves away from the special head the mechanical clamp starts being tensioned in such a way that it acts as a linking element between two previously separated body parts;
- third step: cutting of the clamp, wherein the professional pressures the pair of levers against each other, and said movement activates the cutting functions, thus separating the non-used element from the body of the clamp;
- fourth step: finishing of the ends, wherein the ends of the metal clamp are joined together by means of the intervention of the head snout, resulting in a high quality reliable junction.

Critical analysis of the multi-function pliers: although the positioning, tensioning, folding, cutting and finishing functions of the finished metal clamp are satisfied, restrictive aspects are appreciated, translated into the form of problems specially related to the long time spent in applying and finishing the metal clamp, which time is potentiated by limitations in the accomplishment of the handling functions of the multi-functions pliers during the tensioning step, requiring the segment of the metal clamp to be folded for cutting the excess segment of the metal clamp, and such restrictive condition must be duly satisfied.

### Identification of the problems and the causes thereof:

1. Difficulty in handling the multi-function pliers from the tensioning operation of the constructive disposition of the traction pin, wherein same is fixed to the body of the spindle, thus conceptually forcing the spindle as it is impelled by the rotation of the regulating cylinder to be positioned on the external side of the second lever.
   This condition imposes a difficulty condition to one skilled in the art, since the positioning of the spindle on the external side of the second lever provides the pliers with an excessive length that deprives the subsequent operation of folding the cutting segment of ergonomics, since it should be pointed out that the region of consolidation of the surgical intervention is extremely sensitive.
2. Difficulty in separating the excess segment of the metal clamp already applied, in view of the fact that the time is the critical parameter for completing the surgical intervention, and it is evident that this time is extended by the lack of efficiency when the clamp is cut by the perpendicular position of the cutting blades in relation to the surface of the clamp, thus requiring the application of higher power to cut same.
   This condition may bring about sudden movements and thus lead to the displacement of the recently fixed structures and interfering with the effectiveness of the procedure.
   The negative condition disclosed above is potentiated even more in the region already sensitized by the surgical intervention, wherein any careless action may cause injury in this region, thus compromising the whole surgery procedure.
   Thus, in a remissive way, it is evident that the need for a folding step with alternating movements of the pliers compromises the time required for the consolidation of the finishing procedure of the invasive surgical intervention.
3. Difficulty in handling the levers of the multi-function pliers: it occurs as a function of a final simple plastic design of the levers that results in a part for a compressive movement with one of the hands lacking anatomical conditions thus leading to an additional difficulty in the stable control of the multi-function pliers of the prior art.
4. The tension applied to the clamp is unknown because of the lack of a specific indicator, thus making the application of the clamp in patients suffering from osteoporosis quite delicate in view of the possibility that the bone may break due to excessive tension.

In this way, as prior art we have the document EP 1 731 106 of the same applicant, as specified in the preamble of claim 1.

### PROPOSAL OF THE INVENTION

In view of what was shown in the Background of the Invention section, the inventor has devised a new tool for the application of a metal clamp when a invasive surgical intervention is completed in order to promote the attachment of the affected bone structure, whose innovations eliminate the causes of the problems identified in the multi-function pliers of the prior art.

With relation to the multi-function pliers cited and evidenced in the Background of the Invention section, the inventor has introduced the following innovations:
1. A clamp tensioning device: to prevent the body of the pliers itself from being too long, the new device is provided with functional logics wherein the spindle rotates freely inside a cavity formed in the second lever, remaining definitively inside same, wherein the linear displacement is exclusively effected by an carriage provided with traction pin that, in view of the fact that it is provided with an endless thread through which the spindle passes, runs over the whole extension of the spindle when the spindle is turned, thus impelling the segment of the metal clamp;
2. Device for cutting the segment of the metal clamp: a pair of cutting blades is inserted, each of which at one of the ends of each lever where the cutting head is defined, both of which being obliquely positioned to each other, thus assuring that the simple movement of closing said blades may effect the cutting of the metal segment with little effort and thus eliminating the need of folding movements to break same later on in relation to the metal clamp itself;
3. Dynamometer with applied tensioning power indicator: comprised of a set of a compressive spring inserted inside the second lever below the handle and crossed over by the spindle that, by resisting the displacement of the spindle, shows the instrument nurse in a graduated scale the actual tension applied by the clamp on the bone structure of the cylinder that covers the compressive spring when it goes inside the second lever, which is of relevance in situations where the patient suffers from osteoporosis, for instance.
4. Safety blocking device: its function is to prevent the accidental cutting of the segment of the metal clamp when the dynamometer is "filled up with tension", thus preventing the spindle from being abruptly ejected out of the cavity of the second lever, thus preventing the occurrence of accidents involving both the professional who is handling the pliers and the patient, since the pliers are positioned adjacent to his/her breast when the cutting is carried out.
   In particular, said safety device is mandatory due to the fact that the concept wherein the tensioning of the metal clamp applied by the multi-function shears generates a tensioning condition on the spindle, said tensioning in the form of compression power being transferred to the compressive spring that remains compressed when the optimum compression point of the metal clamp is attained.
   This condition described herein generates an undesired effect that is called "rebound effect", when the excess segment of the metal clamp is cut by the cutting device, which was explained by actual facts and put into practice, that immediately after said cutting the condition of effective tensioning determined by the movement of the carriage provided with traction pin ceases to exist, thus releasing abruptly the whole tensioning power trapped through the compression of the compressive spring, so that the spindle to which the carriage provide with traction pin is associated retreats abruptly and is improperly expelled from inside the cavity of the lever where it is housed, thus hitting the instrument nurse and possibly the patient, thus creating a critical safety condition.
   To solve this problem the inventor has devised the safety lock the operational logics of which is based on the creation of an interference when the cutting device is actuated, in such a way that it is actuated only when a safe condition wherein the rebound effect does not take place, that is, the cutting of the segment of the clamp occurs only when after the energy accumulated in the dynamometer is dissipated by the rotation of the handle for returning the carriage provided with traction pin, and this is effected after the metal clamp is locked as soon as the intended tension is attained.
5. Anatomical configuration of the levers: to facilitate rotational movements for displacing the carriage provided with traction pin of the novel device for tensioning the segment of the metal clamp, as well as to facilitate the compression of the pair of levers when the new device for cutting the segment of the metal clamp is actuated, said levers starting showing "anatomical grasp" shapes.

### DESCRIPTION OF THE FIGURES

To complement the present description in order to get a better understanding of the characteristics of the present invention, a set of drawings is attached hereto, disclosing a primary version of the multi-function pliers anticipated in the state of the art and also in an exemplified but non-limiting way, a first form of the improved embodiment of the same multi-function pliers, evidencing a novel device for tensioning the clamp, cutting device, indicator of the tension applied by the clamp and anatomical shape of its levers and also a second form of accomplishing the same multi-function plies with a constructive variant of the dynamometer as well as a novel safety device that prevents the early cutting of the excess segment of the clamp, wherein:
Figure 1a is a representation illustrating two bone regions and specific situations where the novel shears with tensioner and dynamometer is applied for attaching two parts in a surgical intervention;
Figure 1b is a perspective view representing a multi-function pliers of the prior art;
Figure 2 is a perspective view representing the novel shears with tensioner and dynamometer with both the tensioner and the cutting device claimed herein;
Figure 2a is an elevational view representing the first lever that comprises the novel shears with tensioner and dynamometer with both the tensioner and the cutting device claimed herein;
Figure 2b is a side view representing the first lever that comprises the novel shears with tensioner and dynamometer with both the tensioner and the cutting device claimed herein;
Figure 3a is an orthogonally cut "XX" representation of the first lever that comprises the novel shears with tensioner and dynamometer with both the tensioner and the cutting device claimed herein;
Figure 3b is an orthogonally cut "YY" representation of the first lever that comprises the novel shears with tensioner and dynamometer with both the tensioner and the cutting device claimed herein;
Figure 3c is an orthogonally cut "ZZ" representation of the first lever that comprises the novel shears with tensioner and dynamometer with both the tensioner and the cutting device claimed herein;
Figure 4a is an elevational view representing the second lever that comprises the novel shears with tensioner and dynamometer with both the tensioner and the cutting device claimed herein;
Figure 4b is a side view representing the second lever that comprises the novel shears with tensioner and dynamometer with both the tensioner and the cutting device claimed herein;
Figure 5a is a side view representing the carriage provided with traction pin that comprises the novel shears with tensioner and dynamometer with both the tensioner and the cutting device claimed herein;
Figure 5b is a front view representing the carriage provided with traction pin that comprises the novel shears with tensioner and dynamometer with both the tensioner and the cutting device claimed herein;
Figure 6a is a side view representing the spindle that comprises the novel shears with tensioner and dynamometer with both the tensioner and the cutting device claimed herein;
Figure 6b is a front view representing the spindle that comprises the novel shears with tensioner and dynamometer with both the tensioner and the cutting device claimed herein;
Figure 7a is a perspective view of the clamp in the opened position, to be applied by using the novel shears with tensioner and dynamometer with both the tensioner and the cutting device claimed herein;
Figure 7b is a perspective view of the clamp in the closed position for adjustment, to be applied by using the novel shears with tensioner and dynamometer with both the tensioner and the cutting device claimed herein;
Figure 8a is a representation illustrating the step of positioning the excess segment of the metal clamp close to the cutting head defined in the first lever and fitting its end into the carriage provided with traction pin;
Figure 8b is a representation illustrating the step of tensioning the excess segment of the metal clamp;
Figure 9a is a representation illustrating the step of cutting the excess segment of the metal clamp, immediately after the segment is cut and split;
Figure 9b is a representation illustrating the step of cutting the excess segment of the metal clamp, immediately after the segment is cut and split and the final separation of the shears with tensioner and dynamometer with the tensioner claimed herein of the region where the clamp is applied;
Figure 10a is a magnified representation illustrating in details the device for cutting the excess segment of the metal clamp, immediately after the segment is cut and split;
Figure 10b is a magnified representation illustrating in details the device for cutting the excess segment of the metal clamp, immediately after the segment is cut and split and the final separation of the pliers of the region where the clamp is applied;
Figure 11 is a perspective view representing the novel shears with tensioner and dynamometer evidencing an embodiment of the tension indicator;
Figure 12 is a side view representing the novel shears with tensioner and dynamometer evidencing the embodiment of the dynamometer;
Figure 13 is a top view representing the novel shears with tensioner and dynamometer evidencing an embodiment of the tension indicator;
Figure 14 is a magnified representation illustrating in details the first embodiment of the tension indicator, evidencing its constructive and functional concept;
Figure 15 is a perspective view representing the novel shears with tensioner and dynamometer evidencing an example of the tension indicator;
Figure 16 is a side view representing the novel shears with tensioner and dynamometer evidencing the second embodiment of the tension indicator;
Figure 17a is a magnified representation illustrating in details said example of the tension indicator, evidencing its functional concept, mainly when tension is not applied to compress the metal clamp;
Figure 17b is a magnified representation illustrating in details said example of the tension indicator, evidencing its functional concept, mainly during the application of tension for compressing the metal clamp;
Figure 18 is a magnified representation illustrating in details said example of the device dynamometer, evidencing its parts and functional logics;
Figure 19 is a side view representing the novel shears with tensioner and dynamometer evidencing an embodiment of the safety blocking device, in a condition immediately before the tensioning of the metal clamp;
Figure 20 is a magnified representation illustrating in details the device security constraint, evidencing its parts and functional logics immediately before the tensioning of the metal clamp;
Figure 21 is a side view representing the novel shears with tensioner and dynamometer evidencing an embodiment of the safety blocking device, during the tensioning of the metal clamp; and
Figure 22 is a magnified representation illustrating in details the device security constraint, evidencing its parts and functional logics, during the tensioning of the metal clamp.

### DETAILED DESCRIPTION

The following detailed description should be read and interpreted with reference to the drawings shown herein, all of which are highly diagrammatic, representing a preferred embodiment of the invention, and are not meant to limit the scope of the invention that is limited only by what is disclosed in the set of claims.

### Field of application:

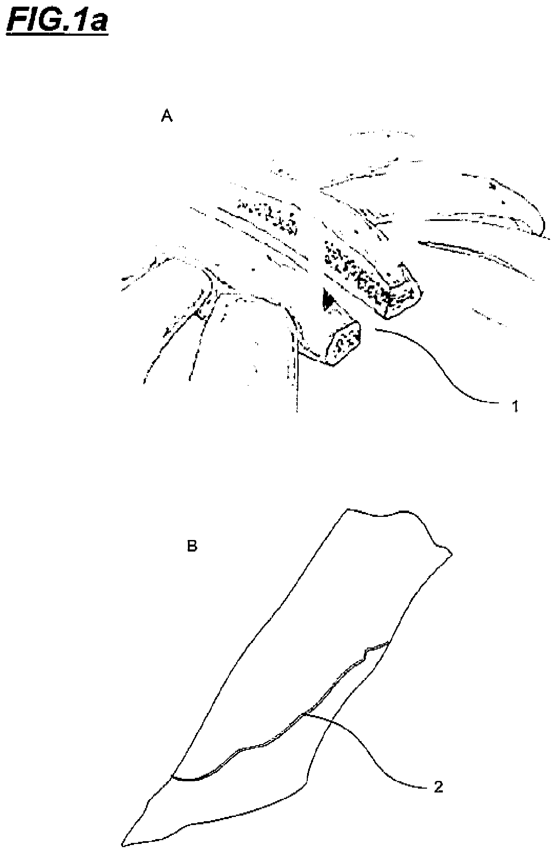

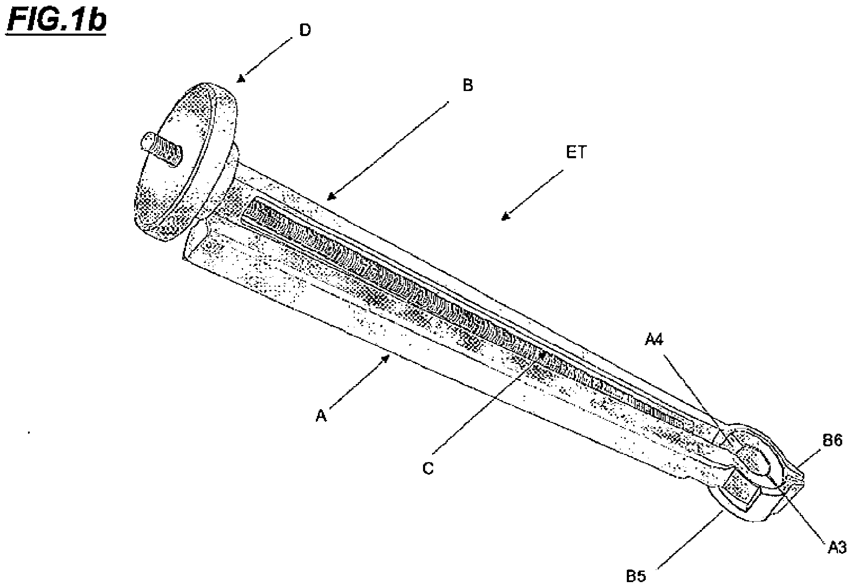

The inventor understands that, for a better understanding of the field of application of the invention, it is pertinent to present in figure 1 an illustrative representation of two bone structures and specific situations wherein specifically the novel shears with tensioner and dynamometer with a tensioner is applied with great advantage according to the point of view of the procedure for attaching two parts together in a surgical intervention.

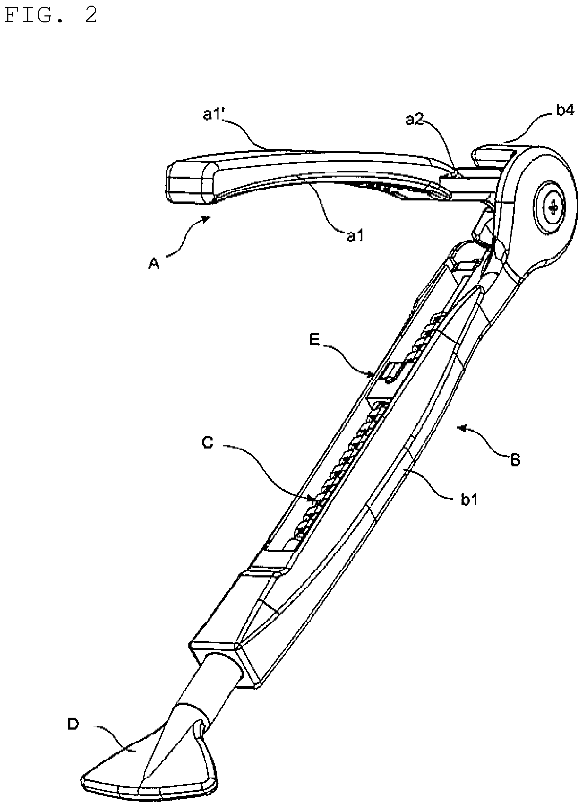

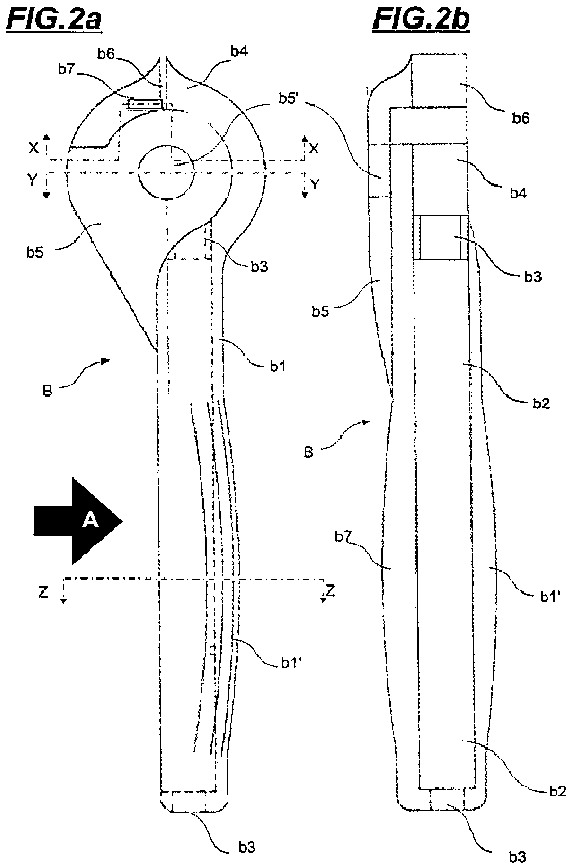

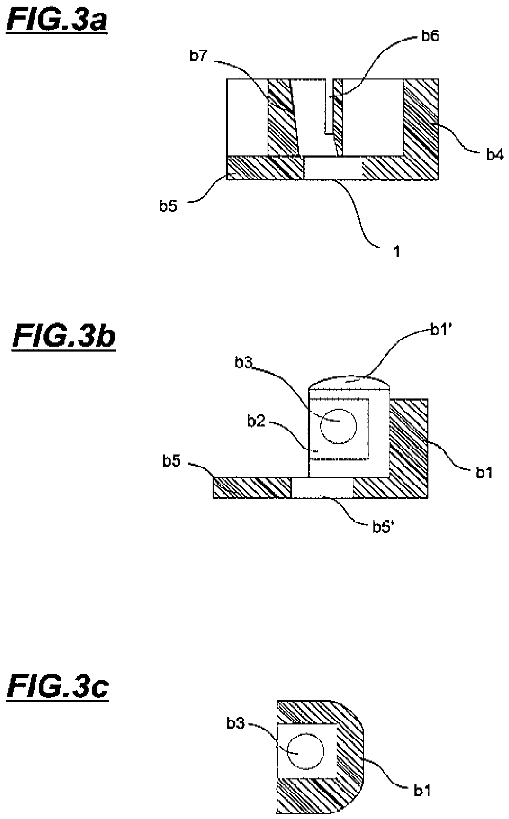

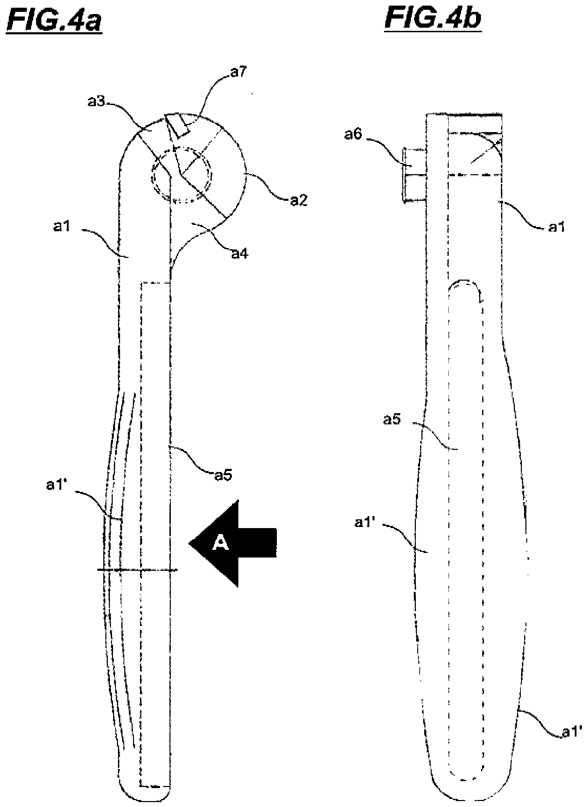

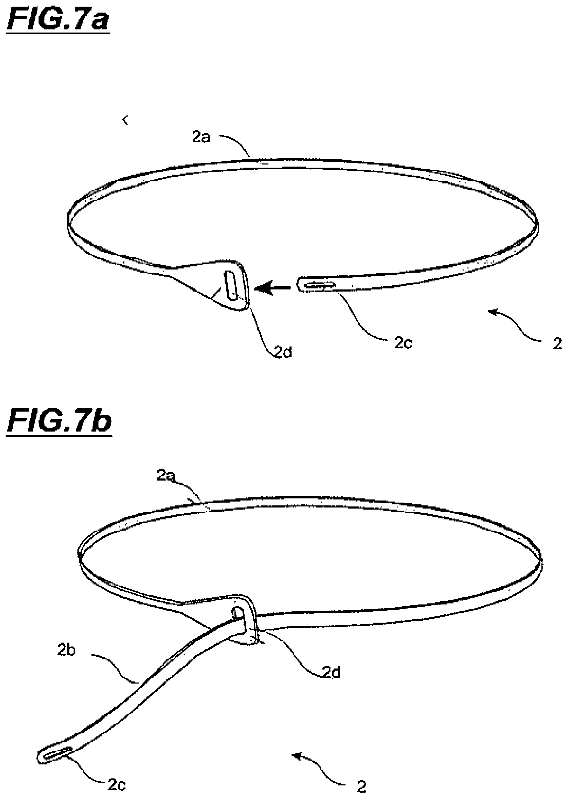

Technological grounds of the multi-function pliers: the inventor understands that for a better understanding of the distinctive characteristics in the present invention it is pertinent to present a embodiment of the original model of multi-function pliers (ET), as can be generically appreciated in figures 1b and 2, comprising the components:
- First lever (A): as depicted in figures 4a and 4b, respectively, it is comprised of a main body (A1), and may present a squared cross-section among others, the function of which is to provide support in the application of a cutting power. The upper end of the main body (A1) has a semi-cylindrical formation (A2) with a structured wall, whose primary function is to fix the second lever (b) in order to form a rotation axle. On its turn, the semi-cylindrical formation (A2) has a slit (A3), the function of which is to apply the segment of the clamp (2) to be compressed (2B), which segment is duly illustrated in figures 7a and 7b, respectively. On its turn, an alignment wall (A4) that delimits an end of the semi-cylindrical formation (A2) is provided, the main function of which is to align the segment (2b) to be compressed.
   Also within the scope of the semi-cylindrical formation (A2), more specifically in relation to the slit (A3), a tooth-shaped shoulder (A5) is provided, the function of which is to facilitate the cutting of the same segment to be compressed (2b).
- Second lever (B): as depicted in figures 2a, 2b; and detailed in figures 3a, 3b and 3c, respectively, it is comprised of a little more specific structure that, similar to the first lever (), has a main body (B1), but it is provided with a housing (82) inside same, the function of which is to provide the assembling and structuring of the spindle (C), the lower end portion of which has a smooth orifice (83) the function of which is to rotate the spindle (C). To complement the upper end portion of the extruded linear profile (B1) a structure called head (84) is defined, which on its turn can also be considered in a semi-cylindrical shape, and is supported by a circular base (B5), the function of which is to receive the assembly of the semi-cylindrical formation (A2) of the first lever ().

As to the upper end position of the head (84) the access snout (B6) is defined, the central portion of which has a slit formation defined therein, the function of which is to apply the segment (2b) of the metal clamp (2) to be compressed together with the slit (A3) of the semi-cylindrical formation (A2) of the first right lever (A).

On the right internal portion of the slit (A3), a tooth-shaped shoulder (B7) is defined, the function of which is to facilitate the cutting of the same segment (2b) of the metal clamp (2) to be compressed by acting together with the tooth (A5) provided for in the internal portion of the slit (A3) defined in the semi-cylindrical formation (A2) of the first lever (A);
- a spindle (c) : as depicted in figure 1, provided with a spindle (C1) having a thread formation all over same, a fitting shoulder being defined on its upper portion, the function of which is to provide a suitable fit for tensioning the segment (2b) of the clamp (2) to be compressed later on.
- Tensioning handle (D): as depicted in figure 1, wherein it is mounted in the lower end of the spindle (C).

### Distinctive features of the invention:

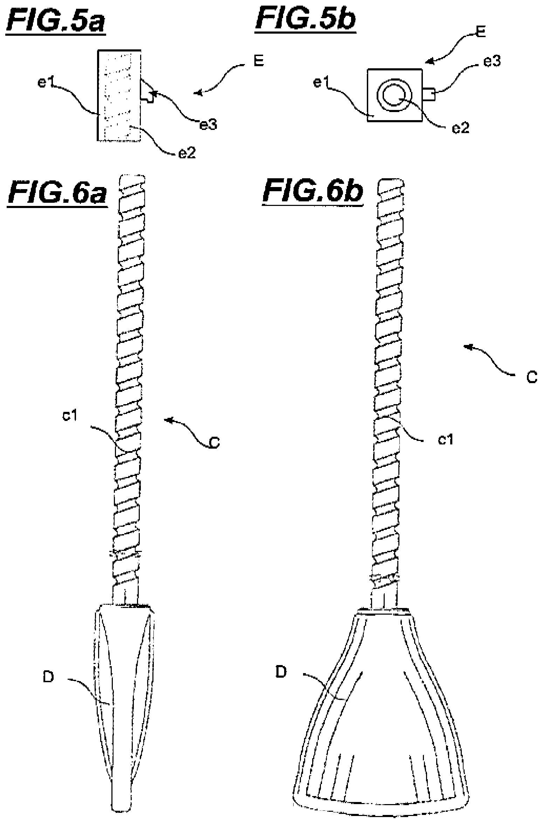

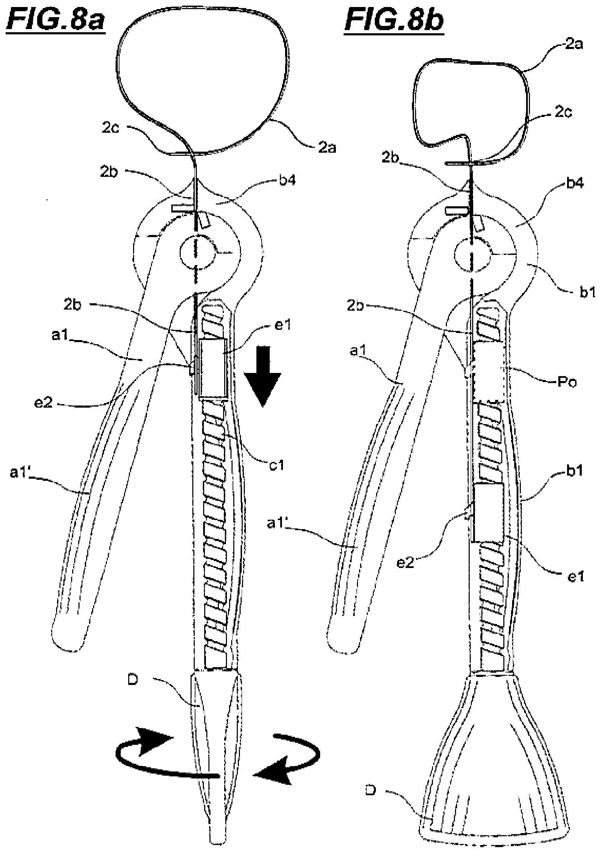

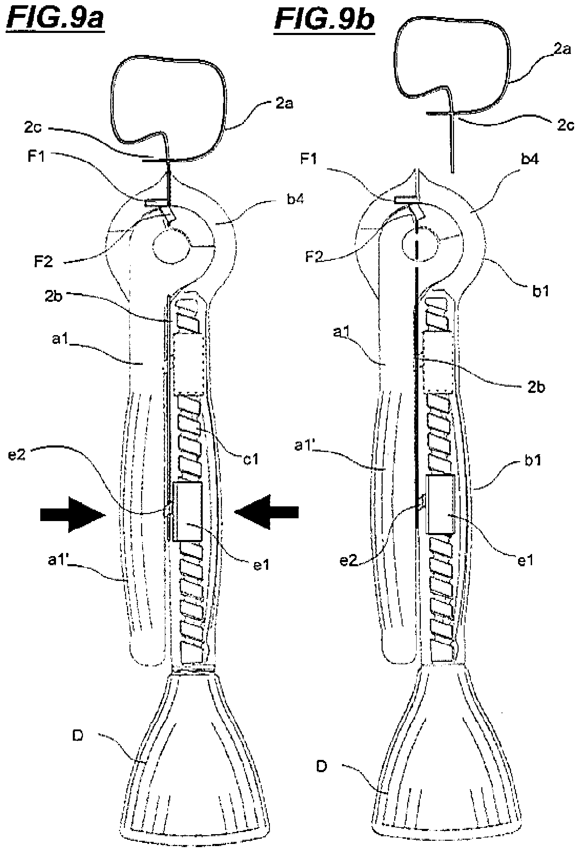

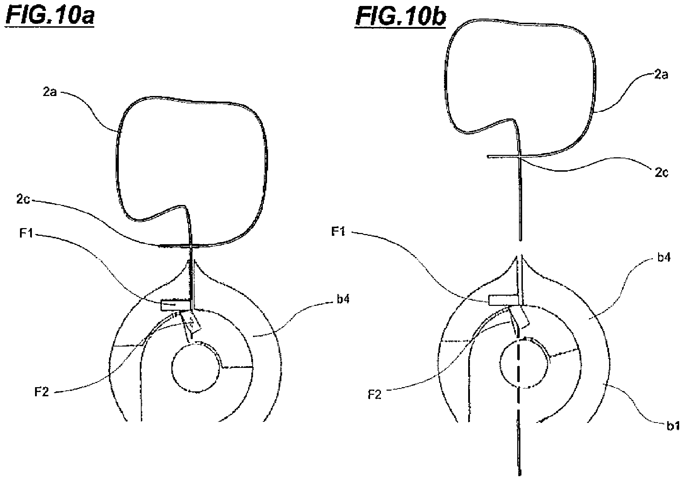

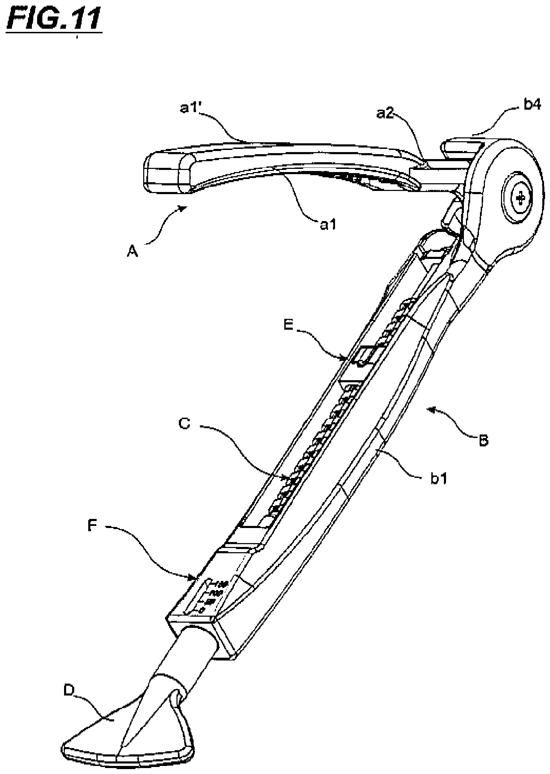

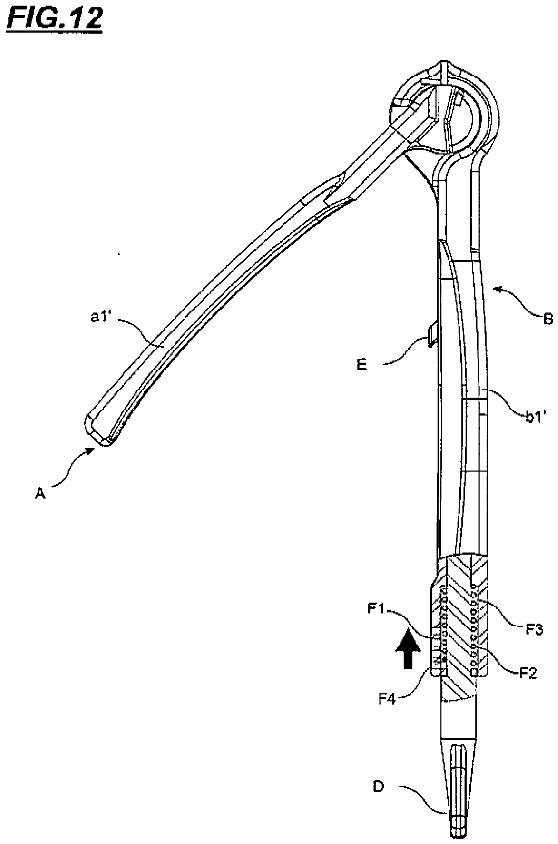

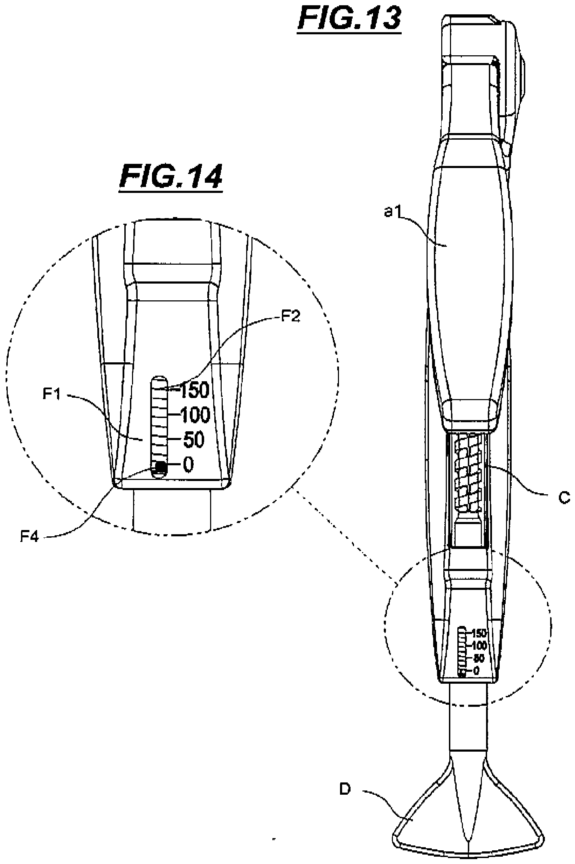

1. Detailed description of the new device for tensioning the metal clamp: comprised of a spindle (C) as depicted individually in figures 6a and 6b, respectively, being comprised of a spindle (C1) that is mounted inside the housing (82) defined in the second lever (8), see figures 8a and 8b and 9a and 9b, respectively, the free end of which passes through the smooth orifice (B3) defined inside the first lever (8) and located inside smooth orifice (83) defined in the upper end region of the main body (81), in such a way that said spindle (C1) rotates freely inside each smooth orifice (83) mentioned hereinabove.
   Also, along the spindle (C1) the carriage provided with traction pin (E) of the segment (2b) of the metal clamp is defined (2), as depicted in figures 5a and 5b, respectively, which carriage is comprised of a monolithic block (E1) having a threaded through-hole (E2) that is designed to be mounted and slid with spindle (C1) of the spindle (C), a hook (C3) that fits into the free end of segment (2b) of the metal clamp (2) also being defined on the upper external face thereof.
   Lastly, a rotatable handle (D) is mounted at the free end of the spindle (C), see figures 6a and 6b, respectively, with the difference that it is anatomically shaped and thus assures an optimum "grasping" condition of the pliers for the instrument nurse when the segment (2b) of the metal clamp (2) is tensioned.
   Also, the functional concept of the new device for tensioning the metal clamp, as depicted in figures 8a and 8b, respectively, may be understood through the operational condition wherein the carriage provided with traction pin (E) is already provided with the segment (2b) of the metal clamp (2) duly fit into the access snout (B6) of the first lever (8) and also with the end of said segment (2b) duly attached to the hook (C3), see figure 8a, wherein the instrument nurse proceeds to rotate the handle (D), in such a way that the body of the spindle (C1) rotates freely, thus moving the carriage provided with traction pin (E) downward, so that in this operation it is evidenced that said spindle (C) remains permanently inside the housing (B2) of the first lever (8), as can be appreciated in figure 8b, so that this condition promotes the adjustment of the body (2a) of the clamp (2).
2. Detailed description of the new device for cutting the segment of the metal clamp: a pair of cutting blades (b7) and (a7) is inserted, as depicted in figures 2a; 2b; 3a; 4a and 4b, respectively, each of which is placed at one of the ends of each of first lever (8) and second lever (A), mainly where the access snout (B6) of the first lever (8) and the slit (A3) of the second lever (A) are defined, respectively, the blade contained in the internal cylinder being obliquely positioned in relation to the blade of the external cylinder, thus applying the cutting pressure on the surface of the clamp gradually and not perpendicularly, just like any scissors.
   Also, the new device for cutting the segment of the metal clamp, as depicted in figures 9a and 9b, respectively, may be understood when the instrument nurse grasps the first lever (B) and the second lever (A) and applies a compression power on both with the main bodies (B1) and (A1), thus closing and narrowing to a point of the segment (2b) of the clamp (2) next to the access snout (B6) of the first lever (B) and the slit (A3) with the consequent compression thereof through the action of the cutting blades (b7) and (a7), the narrowing point being sheared when its cutting faces provide an oblique position between same, thus breaking this segment (2b) apart from the body (2a) of the metal clamp (2), see figures 10a and 10b, respectively.
3. New anatomical configuration of the mobile components: to facilitate the rotation for displacing the carriage provided with traction pin (E) of the novel device for tensioning the segment of the metal clamp, the tensioning handle (D) is provided with a new anatomical shape that assures an optimum "grasping" condition of the pliers when the instrument nurse tensions the segment (2b) of the metal clamp (2).
   Also, to facilitate the compression of the levers (A) and (B), respectively, when the new device for cutting the segment of the metal clamp (2) is actuated, the main bodies (A1) and (B1) of the respective levers are provided with an anatomical shape (A7) and (B7).
4. Detailed description of the dynamometer:
   As depicted in figures 11; 12; 13 and 14, the tension indicator (F) is comprised of a display (F1), located at the lower end of the second lever (8), provided with an oblong slit and tension graduation on one side thereof, an indicating point (F4) being defined next to said slit, said point being marked at the lower end of a compressive spring (F2) mounted on the lower housing (F3) defined in the internal end of the second lever (8) that passes through its inner diameter in the spindle (C) which is also delimited by the tensioning handle (D).

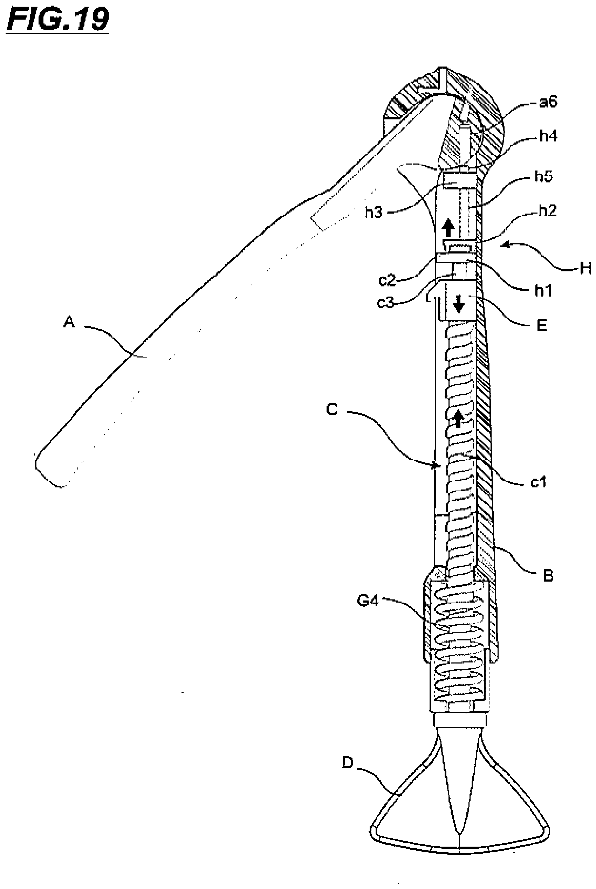

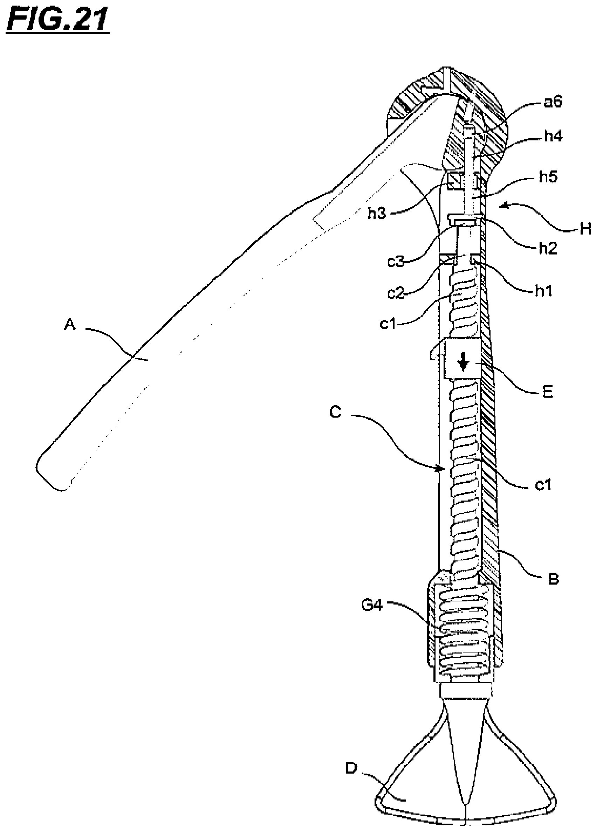

   Also, the functional concept can be better understood by the fact that as the instrument nurse rotates the tensioning handle (D) the device for tensioning the metal clamp (2) is actuated in such a way that the whole spindle (C) together with the handle (D) is impelled, thus compressing the compressive spring (F2) so that the indicating point (F4) is displaced along the display (F1) lining up the tension indicating point with the side graduation of the display (F1) that shows the resistance applied by the spring, indicating the instrument nurse the precise value of the compressive tension that the clamp (2) exerts on the bone structure to be recovered.

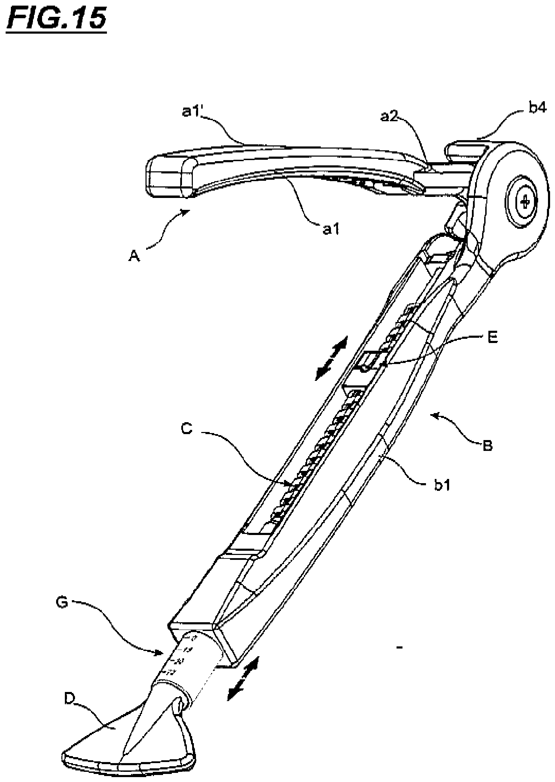

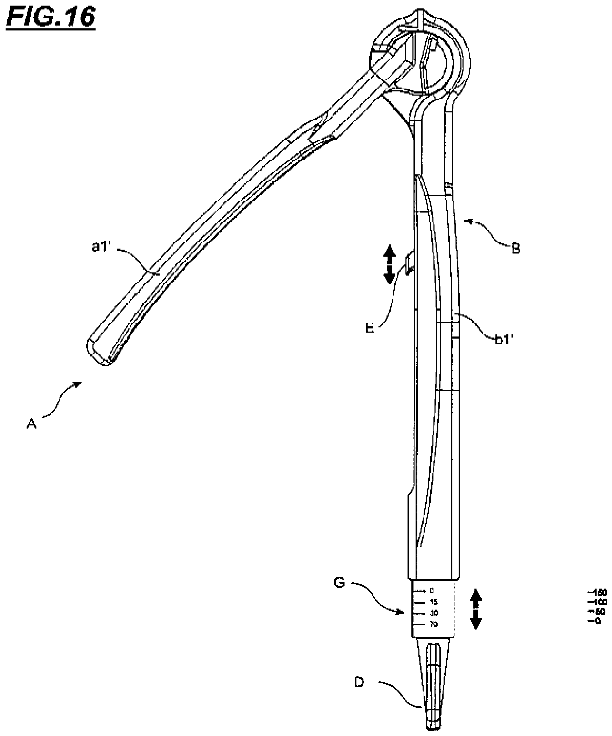

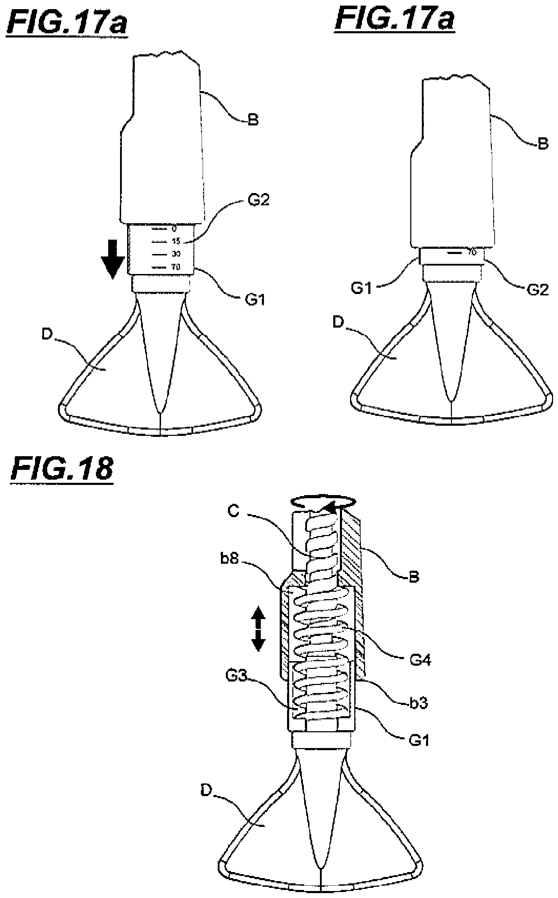

   Figures 15; 16; 17a, 17b and 18 show an example of another tension indicator (G) comprised of a part called indicator (G1) in the shape of a cylindrical bushing, the surface of which has a tension graduation (G2), the inner diameter of said indicator (G1) passing through the spindle (C) and being delimited by the body of the handle (D), the outer diameter of said indicator (G1) also passing adjacent the smooth orifice (b3) of the second lever (B).
   The indicator (G1) also has a housing (G3) the function of which is to accommodate a portion of the body of compressive spring (G4) that on its turn also is mounted with its diameter passing through the spindle (C) and delimited inside the back housing (b8) defined in the back end of the lever (B).
   Also, its concept functional can be better understood by the fact that as the instrument nurse rotates the tensioning handle (D), the device for tensioning the metal clamp (2) is actuated in such a way that the whole spindle (c) together with the handle (D) is displaced towards the front of the housing (b2) of the main body (b1) of the second lever (B), thus compressing the compressive spring (G4) that is restrained by the interference of an end of said spring with the bottom of the housing (G3) of the indicator (G1) and its other end with the bottom of the back housing (b8) of the lever (B). In addition, the displacement of the body in the shape of a bushing of the indicator (G1) passes through the smooth orifice (b3) of the second lever (B), said orifice having a second function to indicate the tension by aligning the tension graduation (G2) of the indicator (G1) with the same and consequently indicating the instrument nurse the precise value of the compressive tension that the clamp (2) exerts on the bone structure to be recovered.
6. Safety blocking device [H] as depicted in figures 19, 20, 21 and 22, respectively, wherein its constructive concept is based on the introduction of a smooth segment of the non-threaded spindle [c2] that passes through a first support [h1] that is fit into the cavity of the second lever [B], the base of the spindle [c3] that is connected to the back portion of the lock base [h2] being defined at the end of said smooth segment of the spindle [c2], a blocking pin [h4] being mounted orthogonally thereto, said pin passing through the opening of the second support [h3] which also is fit into the cavity of the second lever [B]. On its turn, a return spring [h5] passes through the whole body of the blocking pin [h4].

Lastly, a channel [a6] the function of which is to receive the blocking pin [h4] while the clamp [2] is tensioned is defined on the body of the first lever [A], specifically in the region of the semi-cylindrical formation (A2).

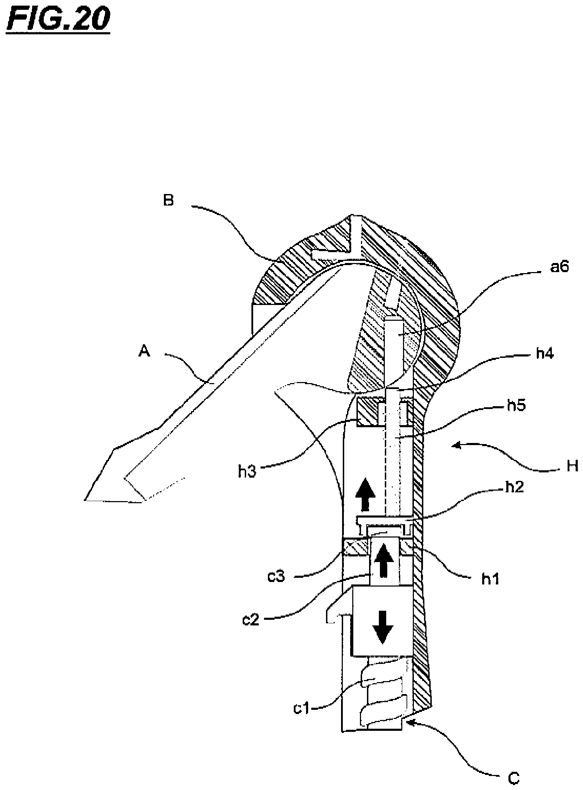

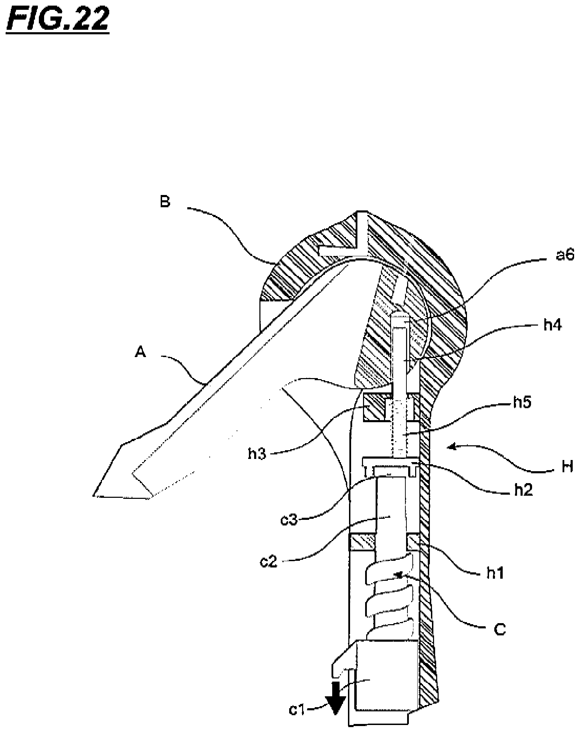

On the other hand, the functional logics of the safety blocking device can be better understood by appreciating figures 20 and 22, respectively, where at first, immediately before the application of tension to the clamp [2], see figure 19, it can be seen that the compressive spring [g4] of the tension indicator is free from any sort of tensioning.

As soon as the instrument nurse starts rotating the handle [D], the spindle [c1] starts actuating the carriage provided with traction pin [E] in such a way that it starts being displaced linearly inside the housing [b2] of the second lever [B], specifically towards the handle [D], whereas the spindle [c1] itself is discretely displaced simultaneously to the front of said housing [b2], in such a way that said displacement causes the consequent displacement of the smooth segment of the spindle [c2] so that the base of the spindle [c3] pushes the base of the lock [h2] and consequently displaces the blocking pin [h4] towards the interior of the channel [a6] defined in the first lever [A], thus compressing the return spring [5], as evidenced in figure 22.

The interference of the blocking pin [h4] inside the channel [a6] defined in the first lever [A] assures that the instrument nurse does not actuate the cutting device improperly, that is, it prevents the levers [a] and [B] respectively from closing.

Thus, with the displacement of the blocking pin [h4] towards the interior of the channel [a6] defined in the first lever [A], this displacement cause a compression of return spring [5] and prevents the levers [a] and [B] respectively from closing.

The embodiments described in this detailed section of the invention "shears with tensioner and dynamometer for applying a metal clamp when an invasive surgical intervention is completed wherein the fixation of the affected body structure is required" are provided only as an example. Alterations, modifications and variations can be made through other particular embodiments by those skilled in the art without, however, departing from the scope as defined by the attached claims.

## Claims

1. Multi-function pliers with tensioner and dynamometer comprised of a first lever (A) made out of a main body (A1) the upper end of which having a semi-cylindrical formation (A2) with a structural wall, to which a second lever (B) is mounted, the semi-cylindrical formation (A2) having a slit (A3) where the segment (2b) of a clamp (2) is applied, an alignment wall (A4) that delimits an end of the semi-cylindrical formation (A2) being provided therein, said slit (A3) having a tooth (A5); said second lever (B), comprised of a main body (B1) having a housing (B2) inside same where a spindle (C) is mounted through a smooth orifice (B3), a head (B4) being defined on the upper end portion of the body having an extruded linear profile (B1) that supports a circular base (B5) that receives the semi-cylindrical formation (A2), an access snout (B6) being defined on the upper end position of the head (B4) the central portion of which defines a slit formation where the segment (2b) of a metal clamp (2) is applied, and a tensioning handle (D) is defined on the lower end portion of the spindle (C); a device for cutting the segment of the composed metal clamp (2) by a pair of cutting blades (b7) and (a7), each of them at one of the ends of each of said first lever (A) and second lever (B), mainly where the access snout (B6) of the second lever (B) and the slit (A3) of the first lever (A), respectively, are defined; a device for tensioning said metal clamp (2) comprised of said spindle (C) defined by a threaded longitudinal shaft (C1) mounted inside the housing (B2) of the second lever (B) the free end of which passes through the smooth orifice (B3) defined in the inner end of the second lever (B) and located inside the smooth orifice (B3) defined on the upper end portion of the main body (B1); **characterized by** a carriage provided with traction pin (E) of the segment (2b) of the metal clamp (2) being defined along the spindle (C1) which carriage is comprised of a monolithic block (E1) having a threaded through-hole (E2) mounted next to the threaded longitudinal shaft (C1) of the spindle (C) the upper external face of which defines a hook (e3), by said handle (D) being an anatomical rotatable handle (D) mounted at the free end of the spindle (C); by a tension indicator (F) of the tension of said clamp (2), comprised of a display (F1) located at the lower end of the second lever (B), provided with an oblong slit and tension graduation on one of its sides, so that in the region of said slit an indicating point (F4) is defined that is marked in the lower end of a compressive spring (F2) mounted on the lower housing (F3) defined in the internal end of said second lever (B) and passing its inner diameter through the spindle (C) and also delimited by the tensioning handle (D); and by a safety blocking device (H) comprised of a smooth segment (C2) of the spindle (C) without any thread extending from the spindle (C) that passes through a first support (h1) that fits into the cavity of the second lever (B), the end of said smooth segment (C2) of the spindle (C) forming a base of the spindle (C3) connected to the back portion of the base of a lock (h2), a blocking pin (h4) that passes through the opening of a second support (h3) fit into the cavity of the second lever (B) being orthogonally mounted on the front portion thereof, a return spring (h5) passing through the whole body of the blocking pin (h4), a channel (a6) also being defined in the region of the semi-cylindrical formation (A2) of the first lever (A), the displacement of said blocking pin (h4) towards the interior of said channel (a6) causes a compression of said return spring (h5) and prevents said levers (A) and (B) from closing.

## Patentansprüche

1. Die Multifunktionszange mit Spannvorrichtung und Kraftmesser umfasst einen ersten Hebel (A) bestehend aus einem Grundkörper (A1), dessen oberes Ende eine halbzylindrische Form (A2) mit einer tragenden Wand hat, an der ein zweiter Hebel (B) befestigt ist. Das halbzylindrisch geformte Teil (A2) hat einen Spalt (A3), in dem ein Segment (2b) einer Klemmvorrichtung (2) angebracht ist, eine Wand zur Ausrichtung (A4), die ein Ende innerhalb der halbzylindrischen Form (A2) festlegt, worin der besagte Spalt (A3) vorgesehen ist, der einen Zahn (A5) besitzt. Der erwähnte zweite Hebel (B) besteht aus einem Grundkörper (B1), in dem sich ein Gehäuse befindet. In diesem Gehäuse ist eine Spindel (C) eingebaut, die durch eine glatte Öffnung (B3) fährt. Das Kopfteil (B4) am oberen Ende des Grundkörpers besitzt ein stranggepresstes lineares Profil (B1), das eine kreisrunde Grundplatte (B5) unterstützt, die die halbzylindrische Form (A2) aufnimmt. Das obere Ende des Kopf teils (B4) ist in Form einer zugreifenden Schnauze (B6) ausgebildet, deren zentraler Teil die Form eines Spaltes hat, an dem das Segment (2b) einer Klemme (2) aus Metall angebracht ist. Ein Spanngriff (D) ist am unteren Ende der Spindel (C) angebracht; eine Vorrichtung zum Schneiden des Segments der zusammengesetzten Metallklemme (2) mit zwei Klingen (b7) und (a7), die an jeweils einem Ende des erwähnten ersten Hebels (A) und des zweiten Hebels (B) angebracht sind und zwar dort, wo die Schnauze (B6) an dem zweiten Hebel (B) und der Spalt (A3) an dem ersten Hebel (A) ausgebildet sind. Eine Spannvorrichtung der erwähnten Metallklemme (2) bestehend aus der besagten Spindel (C), einer längslaufende Gewindestange (C1), die innerhalb des Gehäuses (B2) des zweiten Hebels (B) montiert ist, deren freies Ende durch die glatte Öffnung (B3) am inneren Ende des zweiten Hebels (B) fährt und die sich innerhalb der glatten Öffnung (B3) befindet, die am oberen Endstück des Grundkörpers (B1) ausgebildet ist.
Charakteristisch ist ein Schlitten ausgerüstet mit einem Mitnehmerbolzen (E) des Segments (2b) der Metallklemme (2), der längs der Spindel (C1) angebracht ist. Dieser Schlitten besteht aus einem monolithischen Block (E1), der ein durchgehendes Gewinde (E2) trägt und neben der längslaufenden Gewindestange (C1) der Spindel (C) angebracht ist und dessen obere Außenseite einen Haken (e3) bildet.
Besagter Spanngriff (D), der gedreht und so anatomisch angepasst werden kann, ist am freien Ende der Spindel (C) ein Spannungsanzeiger (F) angebracht, der die Spannung der besagten Klemme (2) anzeigt. Er besteht aus einer Anzeigevorrichtung (F1) am unteren Ende des zweiten Hebels (B), ausgestattet mit einem länglichen Spalt mit einer Gradeinteilung auf einer Seite, so dass an dem besagten Spalt ein Zeigerpunkt (F4) festgelegt ist, der am unteren Ende einer Druckfeder (F2) markiert ist, die auf dem unteren Gehäuse (F3) montiert ist, festgelegt am inneren Ende des besagten zweiten Hebels (B), durch dessen Innendurchmesser die Spindel (C) läuft und auch von dem Spannhebel (D) sowie durch eine blockierende Sicherheitsvorrichtung (H) begrenzt wird, bestehend aus einem gewindelosen Teil (C2) der Spindel (C), einer Verlängerung der Spindel (C), die durch einen ersten Lagerblock (h1) geht und in eine Vertiefung des zweiten Hebels (B) passt; das Ende des besagten glatten Teils (C2) der Spindel (C), das die Basis der Spindel (C3) bildet und mit dem hinteren Teil eines Schlosses (h2) verbunden ist, ein Sperrstift (h4), der durch eine Öffnung eines zweiten Lagerblocks (h3) geht, der in eine Vertiefung des zweiten Hebels (B) passt und rechtwinklig auf dessen Vorderseite montiert ist. Eine Rückholfeder (h5) geht durch den ganzen Körper des Sperrstifts (h4) hindurch, durch einen Kanal (a6), der auch im Bereich mit der halbzylindrischen Form (A2) des ersten Hebels (A) vorgesehen ist. Die Verschiebung des besagten Sperrstifts (h4) ins Innere des besagten Kanals (a6) gewirkt das Zusammendrücken der erwähnten Rückholfeder (h5) und verhindert, dass die besagten Hebel (A) und (B) sich schließen.

## Revendications

1. Pinces multifonctions à leviers et dynamomètre comprenant un premier levier (A) formant le corps principal (A1) dont l'extrémité supérieure présente une forme de semi-cylindrique (A2) avec une paroi structurale, sur laquelle un deuxième levier (B) a été monté, et la forme semi-cylindrique (A2) contient une fente (A3) où le segment (2b) d'une bride (2) est relié, pourvu d'une cloison d'alignement (A4) qui délimite sur une extrémité la forme semi-cylindrique (A2) ; et cette fente (A3)est pourvue d'une dent(A5); un deuxième levier (B), est composé par un corps principal(B1)avec un boîtier (B2)dans lequel se trouve un axe de rotation(C) monté au travers d'un orifice lisse (B3), où passe une tête (B4) définie sur la partie supérieure de l'extrémité du corps qui présente un profil linéaire extrudé(B1)qui supporte une base circulaire(B5)ainsi que la forme semi-cylindrique (A2), un museau d'accès (B6) est présent sur la position la plus haute de la tête (B4) et la portion centrale de celle-ci comprend une fente où se trouve le segment(2b) d'une bride métallique (2) ainsi que le manche comprenant le levier(D)qui se présente sur la portion inférieure de l'axe de rotation(C); un dispositif pour couper le segment de la pince métallique (2) au moyen d'une paire de lames de découpe (b7) et (a7), chacune d'elles se présentant à chacune des extrémités du premier levier (A) et du deuxième levier (B), principalement à l'emplacement du museau d'accès (B6)du deuxième levier (B) et la fente(A3) du deuxième levier (A), respectivement ;un dispositif pour tendre ladite bride métallique(2)comprenant l'axe de rotation(C) défini comme axe longitudinal fileté (Cl) monté dans le boîtier (B2) du deuxième levier (B) dont l'extrémité libre passe au travers de l'orifice lisse (B3) défini dans l'intérieur de l'extrémité du deuxième levier (B) et placé à l'intérieur de l'orifice lisse (B3)
défini dans la portion supérieure du corps principal[B1); **caractérisé par** un chariot équipé d'une goupille de traction (E) du segment (2b) de la bride métallique (2) définie au long de l'axe fileté (C1) avec le chariot placé dans le bloc monolithique(E1) comprenant un orifice de passage fileté (E2) monté aux côtés de l'axe longitudinal fileté (C1) de l'axe de rotation (C) dont la face supérieure externe comprend un crochet (e3), par ce manche (D) qui anatomiquement rotatif monté sur l'extrémité libre de l'axe de rotation (C); par un tensiomètre (F) indiquant la tension de la bride (2), et comprend un ensemble (F1) placé à l'extrémité inférieure du deuxième levier (B), qui présente une fente oblongue et la graduation de la tension sur un de ses côtés, c'est donc dans la zone de cette fente qu'il y a un point indicateur(F4)marqué sur l'extrémité inférieure du ressort de compression (F2) monté sur la partie inférieure du boîtier (F3) défini dans l'extrémité interne de ce deuxième levier (B) et passant son diamètre intérieur au travers de l'axe de rotation(C) et également délimité par le manche tendeur (D) ; et par un dispositif de blocage de sécurité (H) comprenant un segment lisse(C2) de l'axe de rotation (C) sans filetage au-delà de l'axe de rotation (C) qui passe au travers d'un premier support (h1) qui s'imbrique dans la cavité du deuxième levier (B), et l'extrémité de ce segment lisse(C2) de l'axe de rotation (C) forme la base de l'axe fileté (C3) relié à la partie arrière de la base du blocage (h2), avec une goupille (h4) qui passe au travers de l'ouverture d'un second support (h3) imbriqué dans la cavité du deuxième levier (B)monté de façon octogonale sur sa partie frontale, il y a un ressort de retour (h5) qui passe au travers du corps de la goupille de blocage (h4), une rainure (a6) est étalement présente dans la zone de la forme semi-cylindrique (A2) du premier levier (A), le déplacement de cette goupille de blocage (h4) vers l'intérieur de cette rainure (a6) causes une compression dudit ressort de retour(h5) et évite que ces leviers (A) et (B) ne se referment.
